(19) Europäisches Patentamt
European Patent Office
Office européen des brevets

(11) **EP 4 763 187 A1**

(12) **EUROPEAN PATENT APPLICATION**
published in accordance with Art. 153(4) EPC

(43) Date of publication:
**24.06.2026  Bulletin 2026/26**

(21) Application number: 24940908.7

(22) Date of filing: **01.11.2024**

(51) International Patent Classification (IPC):
*A61K 8/36* (2006.01)   *A61K 8/67* (2006.01)
*A61K 8/64* (2006.01)   *A61Q 19/02* (2006.01)
*A61Q 19/00* (2006.01)

(52) Cooperative Patent Classification (CPC):
A61K 8/36; A61K 8/64; A61K 8/675; A61Q 19/02

(86) International application number:
**PCT/CN2024/129201**

(87) International publication number:
**WO 2026/060784 (26.03.2026 Gazette 2026/13)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority:  **20.09.2024  CN 202411313668**

(71) Applicants:
• **Yunnan Botanee Bio-Technology Group Co., Ltd.
Kuming, Yunnan 650106 (CN)**
• **Yunnan Characteristic Plant Extraction
Laboratory
Co., Ltd.
Kunming, Yunnan 650106 (CN)**

(72) Inventors:
• **WANG, Yichun
Kunming, Yunnan 650106 (CN)**
• **WANG, Feifei
Kunming, Yunnan 650106 (CN)**

• **QU, Liping
Kunming, Yunnan 650106 (CN)**
• **SHEN, Yanzhen
Kunming, Yunnan 650106 (CN)**
• **WANG, Zuding
Kunming, Yunnan 650106 (CN)**
• **WU, Qiongfen
Kunming, Yunnan 650106 (CN)**
• **YAN, Lijuan
Kunming, Yunnan 650106 (CN)**
• **YANG, Zhaoxiang
Kunming, Yunnan 650106 (CN)**
• **MA, Xiao
Kunming, Yunnan 650106 (CN)**
• **GUO, Zhenyu
Kunming, Yunnan 650106 (CN)**

(74) Representative: **Cabinet Chaillot
16/20, avenue de l'Agent Sarre
B.P. 74
92703 Colombes Cedex (FR)**

(54) **COMPOSITION HAVING WHITENING EFFICACY AND APPLICATION THEREOF IN COSMETICS**

(57)   The present invention relates to a composition with a whitening effect. The composition includes artemisinic acid, and further includes nicotinamide and/or glutathione. According to the present invention, the artemisinic acid is creatively compounded with the nicotinamide and/or the glutathione to inhibit generation or transfer of melanin in all directions at multiple angles through different approaches or mechanisms. The composition can significantly inhibit activity of tyrosinase and inhibit production of the melanin, and various active ingredients are complemented with each other to achieve a synergistic effect. Meanwhile, the composition can also enhance water solubility of the artemisinic acid and reduce local irritant toxicity caused by insolubility of the artemisinic acid to enable the artemisinic acid to have good water solubility and low local irritant toxicity while achieving the whitening effect, thereby promoting deep absorption by the skin.

EP 4 763 187 A1

FIG. 1

**Description**

**CROSS-REFERENCE TO RELARED APPLICATIONS**

This application claims priority to Chinese Patent Application No. 202411313668.1, filed on September 20, 2024, entitled "Composition with whitening effect and application thereof in cosmetics", which is incorporated herein by reference in its entirety.

**TECHNICAL FIELD**

The present application relates to the technical field of cosmetics, and particularly relates to a composition with a whitening effect and an application thereof in cosmetics.

**BACKGROUND**

With the improvement of people's quality of life, increasing attention has been paid to skin care and maintenance, and bright and white skin has been considered a type of beauty. The skin color of normal people is mainly determined by the types and distributions of various pigments in the skin, the thickness of the skin, and the scattering of light on the skin surface, etc. Commonly mentioned color spots and pigmentation are related to melanin. A melanin formation process of the skin includes a series of complex processes, such as migration of melanocytes, division and maturation of the melanocytes, formation of melanosomes, transport of melanin granules, and excretion of the melanin.

Pigmentation is a multifactorial, complex, and subtle physiological process involving interactions of multiple regulatory links. This process is not only affected by intrinsic physiological mechanisms, but also regulated by external environmental factors. From the perspective of the intrinsic mechanisms, the pigmentation is closely related to genetics, hormone levels, and metabolic states, etc. Genetic factors play a fundamental role in pigmentation, and differences in skin color among different populations are largely determined by genetics. Meanwhile, changes in the hormone levels also affect generation and distribution of the pigments. For example, skin changes of pregnant women are a typical example. Moreover, the metabolic states also affect metabolism and excretion of the pigments, thereby affecting skin tone. In terms of external environments, factors, such as ultraviolet radiation, chemical substance stimulation, and inflammation, all have impacts on the pigmentation. Ultraviolet light is one of the main factors leading to the skin pigmentation, and long-term exposure to sunlight darkens the skin. Chemical substances, such as certain drugs and cosmetic ingredients, may also cause the skin pigmentation. Inflammation, to a certain extent, promotes the production and deposition of pigments.

Artemisinic acid is a skin-penetrating sesquiterpenoid small-molecule compound derived from Artemisia annua. As an intermediate product in the preparation of artemisinin, current studies on biological activities of the artemisinic acid are only limited to anti-tumor aspects, and broader studies have not yet been conducted.

SUMMARY

In view of the deficiencies of the prior art, an objective of the present invention is to provide a composition with a whitening effect and an application thereof in cosmetics.

To achieve the objective of the invention, the present invention adopts the following technical solutions.

In a first aspect, the present invention provides a composition with a whitening effect. The composition includes artemisinic acid, and further includes nicotinamide and/or glutathione.

The artemisinic acid has a strong anti-inflammatory property, which is conducive to slowing a skin aging process and improving uneven skin tone and dullness. The artemisinic acid reduces production of melanin by inhibiting a key enzyme in a melanin synthesis process, thereby alleviating skin pigmentation and achieving the whitening effect.

The nicotinamide is an amide derivative of vitamin $B_3$ and a PARP inhibitor, which can inhibit formation of melanocytes and reduce deposition of the melanin. The nicotinamide also inhibits transfer of the melanin to keratinocytes, thereby reducing the deposition of melanin in the stratum corneum and further preventing skin darkening. The nicotinamide has an effect of promoting skin metabolism, which can accelerate the transfer of melanin in the keratinocytes to the stratum corneum, and promote shedding of the stratum corneum. This mechanism is conducive to improving issues, such as skin dullness and color spots, thereby making the skin look brighter. The nicotinamide also has an anti-glycation effect, which is conducive to reducing yellowing caused by protein glycation, thereby brightening the skin tone.

The glutathione is a natural antioxidant widely present in animals and plants, which can effectively reduce stimulation of the melanocytes by ultraviolet light and block formation of the melanin in the body, thereby fundamentally inhibiting or reducing the formation of melanin, preventing the deposition of melanin, and inhibiting appearance of spots and freckles. In addition, the glutathione can also regulate peroxyl free radicals and enhance a sulfhydryl group in blood oxygen, thereby making the skin more delicate, whiter, and smoother.

According to the present invention, the artemisinic acid is creatively compounded with the nicotinamide and/or the glutathione to inhibit generation or transfer of melanin in all directions at multiple angles through different approaches or mechanisms. The composition can significantly inhibit activity of tyrosinase and inhibit production of the melanin, and various active ingredients are complemented with each other to achieve a synergistic effect. Meanwhile, the composition can also enhance water solubility of the artemisinic acid and reduce local irritant toxicity caused by insolubility of the artemisinic acid to enable the artemisinic acid to have good water solubility and low local irritant toxicity while achieving the whitening effect, thereby promoting deep absorption by the skin.

Preferably, the composition includes the artemisinic acid and the nicotinamide, and a mass ratio of the artemisinic acid to the nicotinamide is 1:(1-5), where the (1-5) may be selected from 1, 1.5, 2, 2.5, 3, 3.5, 4, 4.5, and 5, etc., and other specific point values within the numerical range may all be selected, which are not repeatedly described individually herein.

Preferably, the composition includes the artemisinic acid and the glutathione, and a mass ratio of the artemisinic acid to the glutathione is 1:(5-10), where the (5-10) may be selected from 5, 5.5, 6, 6.5, 7, 7.5, 8, 8.5, 9, 9.5, and 10, etc., and other specific point values within the numerical range may all be selected, which are not repeatedly described individually herein.

Preferably, the composition includes the artemisinic acid, the nicotinamide, and the glutathione, and a mass ratio of the artemisinic acid, the nicotinamide, and the glutathione is 1:(1-5):(3-7), where the (1-5) may be selected from 1, 1.5, 2, 2.5, 3, 3.5, 4, 4.5, and 5, etc., the (3-7) may be selected from 3, 3.5, 4, 4.5, 5, 5.5, 6, 6.5, and 7, etc., and other specific point values within the above numerical ranges may all be selected, which are not repeatedly described individually herein.

The present application has also creatively discovered that when the artemisinic acid, the nicotinamide, and the glutathione are combined at a specific ratio, the activity of tyrosinase and the generation of melanin can be inhibited more effectively, thereby achieving a better whitening effect.

In a second aspect, the present invention provides a skin care product. The skin care product includes the composition with a whitening effect described in the first aspect.

Preferably, a mass percentage content of the composition with a whitening effect in the skin care product is 1-20%, for example, 1%, 3%, 5%, 7%, 9%, 11%, 13%, 15%, 17%, or 20%, etc., and other specific point values within the numerical range may all be selected, which are not repeatedly described individually herein.

Preferably, the skin care product further includes any one or a combination of at least two of an antioxidant, a preservative, a humectant, a solubilizer, a chelating agent, an emulsifier, an emollient, a thickener, a surfactant, and a pH regulator.

Preferably, the humectant is selected from any one or a combination of at least two of glycerol, propylene glycol, 1,2-hexanediol, hyaluronic acid, sodium hyaluronate, panthenol, sodium pyrrolidonecarboxylate, polyglycerol, butanediol, dipropylene glycol, sorbitol, polyethylene glycols, Lubrajel oil, biosaccharide gum, pentanediol, betaine, or trehalose.

Preferably, the emollient is selected from any one or a combination of at least two of squalane, meadowfoam seed oil, polydimethylsiloxane, a polydimethylsiloxane cross-linking product, shea butter, jojoba seed oil, macadamia ternifolia seed oil, grape seed oil, olive fruit oil, cetyl palmitate, ethylhexyl palmitate, tocopheryl acetate, hydrogenated polyisobutene, C10-18 fatty acid triglycerides, caprylic/capric triglyceride, cyclohexasiloxane, decamethylcyclopentasiloxane, dimethiconol, caprylyl methicone, dicaprylyl carbonate, isododecane, or pentaerythityl tetraisostearate.

Preferably, the skin care product includes a toner, an essence, an emulsion, a cream, a gel, a facial mask, or a facial cleanser.

Compared with the prior art, the present invention has the following beneficial effects.

According to the present invention, the artemisinic acid is creatively compounded with the nicotinamide and/or the glutathione to inhibit generation or transfer of melanin in all directions at multiple angles through different approaches or mechanisms. The composition can significantly inhibit activity of tyrosinase and inhibit production of the melanin, and various active ingredients are complemented with each other to achieve a synergistic effect. Meanwhile, the composition also solves the problem of poor water solubility of the artemisinic acid and reduces toxicity of the artemisinic acid to enable the artemisinic acid to have good water solubility and low toxicity while achieving the whitening effect, thereby promoting deep absorption by the skin.

**BRIEF DESCRIPTION OF THE DRAWINGS**

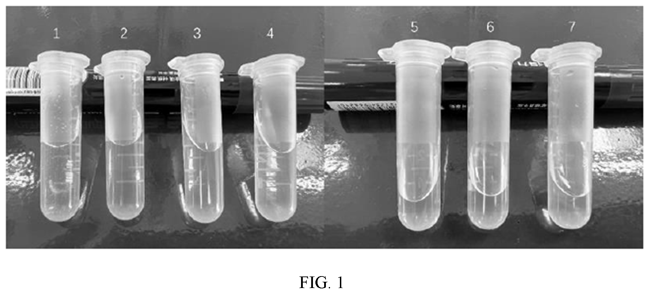

FIG. 1 shows water solubility test results, where 1 refers to an artemisinic acid aqueous solution, 2 refers to an artemisinic acid solution in dimethyl sulfoxide (DMSO), 3 refers to a nicotinamide aqueous solution, 4 refers to a glutathione aqueous solution, 5 refers to an aqueous solution of a composition of Example 5, 6 refers to an aqueous solution of a composition of Example 7, and 7 refers to an aqueous solution of a composition of Example 1.

**DESCRIPTION OF THE EMBODIMENTS**

The technical solutions of the present invention are further illustrated below through specific embodiments.

Those skilled in the art should understand that examples described are merely to facilitate an understanding of the present invention and should not be regarded as specific limitations to the present invention.

In the following examples, unless otherwise specified, both reagents and consumable materials used are purchased from conventional reagent manufacturers in the art. Unless otherwise specified, both experimental methods and technical means used are conventional methods and means in the art.

Artemisinic acid, nicotinamide, and glutathione involved below are all calculated based on actual contents of functional ingredients in commercially available raw materials, and the commercially available raw materials also selectively include any one or a combination of at least two of a solvent, a filler, a diluent, a stabilizer, a pH regulator, an antibacterial agent, am antioxidant, or an impurity within a permissible range.

The artemisinic acid involved in the following examples, comparative examples, application examples, and comparative application examples is a product of model DQ0048 purchased from Desite Biotechnology Co., Ltd.

Sodium hyaluronate is a product of model S0780000 purchased from Merck Sigma-Aldrich.

Carbomer 980 is a product of model YS177293 purchased from Solarbio Science & Technology Co., Ltd.

Butyrospermum parkii butter is purchased from Baichuan Biotechnology (Shanxi) Co., Ltd., with a model of 24-06-2026.

Polydimethylsiloxane is purchased from Shanghai Macklin Biochemical Technology Co., Ltd., with a model of D849784.

PEG-60 hydrogenated castor oil is purchased from Shanghai Macklin Biochemical Technology Co., Ltd., with a model of E875014.

An Artemisia annua extract is prepared by the following method: adding 15 times (m/v) of 95% ethanol into Artemisia annua, performing ultrasonic extraction twice for 1.5 h each time, performing filtration, combining extracts, and performing concentration and freeze-drying to obtain the Artemisia annua extract.

Example 1

This example provides a composition with a whitening effect, which includes, in parts by weight, the following effective ingredients: 1 part of artemisinic acid, 3 parts of nicotinamide, and 5 parts of glutathione.

Example 2

This example provides a composition with a whitening effect, which includes, in parts by weight, the following effective ingredients: 1 part of artemisinic acid, 2 parts of nicotinamide, and 6 parts of glutathione.

Example 3

This example provides a composition with a whitening effect, which includes, in parts by weight, the following effective ingredients: 1 part of artemisinic acid, 4 parts of nicotinamide, and 4 parts of glutathione.

Example 4

This example provides a composition with a whitening effect, which includes, in parts by weight, the following effective ingredients: 1 part of artemisinic acid and 2 parts of nicotinamide.

Example 5

This example provides a composition with a whitening effect, which includes, in parts by weight, the following effective ingredients: 1 part of artemisinic acid and 3 parts of nicotinamide.

Example 6

This example provides a composition with a whitening effect, which includes, in parts by weight, the following effective ingredients: 1 part of artemisinic acid and 8 parts of glutathione.

Example 7

This example provides a composition with a whitening effect, which includes, in parts by weight, the following effective ingredients: 1 part of artemisinic acid and 5 parts of glutathione.

Example 8

This example provides a composition with a whitening effect, which includes, in parts by weight, the following effective ingredients: 1 part of artemisinic acid, 1 part of nicotinamide, and 1 part of glutathione.

Example 9

This example provides a composition with a whitening effect, which includes, in parts by weight, the following effective ingredients: 3 parts of artemisinic acid, 1 part of nicotinamide, and 10 parts of glutathione.

Example 10

This example provides a composition with a whitening effect, which includes, in parts by weight, the following effective ingredients: 3 parts of artemisinic acid and 1 part of nicotinamide.

Example 11

This example provides a composition with a whitening effect, which includes, in parts by weight, the following effective ingredients: 1 part of artemisinic acid and 1 part of glutathione.

Comparative Example 1

This comparative example provides a composition with a whitening effect, which includes, in parts by weight, the following effective ingredients: 3 parts of nicotinamide and 5 parts of glutathione.

Comparative Example 2

This comparative example provides a composition with a whitening effect, which is different from Example 1 only in that the artemisinic acid is replaced with an equal mass of artemisinin.

Comparative Example 3

This comparative example provides a composition with a whitening effect, which is different from Example 1 only in that the artemisinic acid is replaced with an equal mass of an Artemisia annua extract.

Comparative Example 4

This comparative example provides a substance with a whitening effect, which includes a single effective ingredient: artemisinic acid.

Comparative Example 5

This comparative example provides a substance with a whitening effect, which includes a single effective ingredient: nicotinamide.

Comparative Example 6

This comparative example provides a substance with a whitening effect, which includes a single effective ingredient: glutathione.

Application Example 1

This application example provides a cream with a whitening effect, which has a formulation as follows.

| Group | Raw material name | Mass ratio |
|---|---|---|
| A | Water | To 100 |
| | Glycerol (humectant) | 5.00 |
| | Sodium hyaluronate | 0.05 |
| | Carbomer (thickener) | 0.20 |
| B | Butyrospermum parkii butter | 2.00 |
| | Glyceryl stearate (emulsifier) | 1.50 |
| | Polydimethylsiloxane (emollient) | 1.00 |
| C | Aminomethyl propanol (pH regulator) | 3.00 |
| D | P-hydroxyacetophenone (antioxidant) | 0.50 |
| | 1,2-hexanediol (humectant) | 1.00 |
| E | Composition of Example 1 | 3.00 |

A preparation method includes:

(1) sequentially adding raw materials A into an emulsification pot, heating up to 80°C, and performing homogenization and stirring for uniform dissolution;
(2) heating raw materials B to 80°C for dissolution, and stirring the same for uniform dispersion;
(3) adding C into a stirring pot for uniform stirring, and performing homogenization and stirring for uniform dissolution;
(4) heating raw materials D to 60°C for dissolution until a transparent state is achieved, adding the same into the emulsification pot for uniform dispersion, and then cooling to 40°C; and
(5) uniformly dispersing raw material E, adding the same into the stirring pot for uniform dispersion, and then cooling to 20°C to obtain the whitening cream.

Application Example 2

This application example provides a toner with a whitening effect, which has a formulation as follows.

| Group | Raw material name | Mass ratio |
|---|---|---|
| A | Water | To 100 |
| | Glycerol (humectant) | 5.00 |
| | Sodium hyaluronate (humectant) | 0.05 |
| B | P-hydroxyacetophenone (antioxidant) | 0.50 |
| | 1,2-hexanediol (humectant) | 1.00 |
| C | Composition of Example 1 | 3.00 |
| D | PEG-60 hydrogenated castor oil (solubilizer) | 0.02 |

A preparation method includes:

(1) sequentially adding raw materials A into a stirring pot, heating up to 60°C, and performing homogenization and stirring for uniform dissolution;
(2) heating raw materials B to 60°C for dissolution until a transparent state is achieved, adding the same into the stirring pot for uniform dispersion, and then cooling to 40°C;
(3) adding C into the stirring pot for uniform stirring; and
(4) adding and uniformly stirring raw material D, and then cooling to 37°C or below to obtain the whitening toner.

Application Example 3

This application example provides an emulsion with a whitening effect, which has a formulation as follows.

| Group | Raw material name | Mass ratio |
|---|---|---|
| A | Water | To 100 |
| | Glycerol (humectant) | 5.00 |
| | Sodium hyaluronate (humectant) | 0.05 |
| | Disodium EDTA (chelating agent) | 0.02 |
| B | Glyceryl stearate (emulsifier) | 1.50 |
| | Polydimethylsiloxane (emollient) | 1.00 |
| C | Aminomethyl propanol (pH regulator) | 3.00 |
| D | 1,2-hexanediol (humectant) | 1.00 |
| | Composition of Example 1 | 3.00 |

A preparation method includes:

(1) sequentially adding raw materials A into an emulsification pot, heating up to 80°C, and performing homogenization and stirring for uniform dissolution;
(2) heating raw materials B to 80°C for dissolution and uniform dispersion, and then cooling to 60°C;
(3) adding C into the emulsification pot for uniform stirring; and
(4) adding and uniformly stirring raw materials D, and then cooling to 37°C or below to obtain the whitening emulsion.

Test Example 1

This test example determines tyrosinase inhibition effects of the compositions prepared in Examples 1-11 and Comparative Examples 1-6.
With L-tyrosine as a substrate, an experimental group, a blank group, and a positive control group were set.
Experimental group: 100 $\mu$L of the L-tyrosine (0.5 mg/mL), 50 $\mu$L of tyrosinase (200 U/mL), and 50 $\mu$L of aqueous solutions (5 mg/mL) of the compositions prepared in Examples 1-11 and Comparative Examples 1-6 were sequentially added into a 96-well plate.
Blank group: 100 $\mu$L of the L-tyrosine (0.5 mg/mL), 50 $\mu$L of tyrosinase (200 U/mL), and 50 $\mu$L of a PBS buffer were sequentially added into a 96-well plate.
Positive control group: 100 $\mu$L of the L-tyrosine (0.5 mg/mL), 50 $\mu$L of tyrosinase (200 U/mL), and 50 $\mu$L of an aqueous solution (5 mg/mL) of $\alpha$-arbutin were sequentially added into a 96-well plate.
After reactions in the dark for 30 min, the plates were placed into a microplate reader to measure absorbance values at 475 nm, and tyrosinase inhibition rates were calculated. Results are shown in Table 1.

Table 1

| Group | Tyrosinase inhibition rate (%) |
|---|---|
| Positive control group | 96.76 |
| Example 1 | 99.80 |
| Example 2 | 98.00 |
| Example 3 | 96.35 |
| Example 4 | 89.76 |
| Example 5 | 91.64 |
| Example 6 | 86.42 |
| Example 7 | 83.87 |
| Example 8 | 93.80 |
| Example 9 | 83.60 |
| Example 10 | 83.43 |
| Example 11 | 82.32 |

(continued)

| Group | Tyrosinase inhibition rate (%) |
|---|---|
| Comparative Example 1 | 78.50 |
| Comparative Example 2 | 55.80 |
| Comparative Example 3 | 72.30 |
| Comparative Example 4 | 55.64 |
| Comparative Example 5 | 67.34 |
| Comparative Example 6 | 69.76 |

From data in Table 1, it can be seen that the tyrosinase inhibition rates of the compositions prepared in Examples 1-11 are all above 80%, indicating that the compositions involved in the present invention can effectively inhibit the activity of tyrosinase.

By comparing Example 1, Example 5, and Example 7 with Comparative Example 1, it can be found that the combinations of the artemisinic acid, the nicotinamide, and the glutathione play a synergistic role in inhibiting the activity of tyrosinase.

By respectively comparing Example 1 with Examples 8-9, comparing Examples 4-5 with Example 10, and comparing Examples 6-7 with Example 11, it can be found that when the artemisinic acid, the nicotinamide, and the glutathione in the compositions are selected at specific ratios, the tyrosinase inhibition rate can be further increased.

By comparing Example 1 with Comparative Examples 2-3, it can be found that compared with the artemisinin and the Artemisia annua extract, the artemisinic acid used can better inhibit the activity of tyrosinase.

Test Example 2

This test example determines cytotoxicity of the compositions prepared in Examples 1-3, Examples 5-6, and Comparative Example 4.

B16F10 cells in a good growth state were selected, digested, and added into a DMEM culture medium containing 10% of FBS to prepare a cell suspension, and the cells were inoculated into a 96-well plate at a concentration of $5 \times 10^4$ cells/mL, placed in a 5% $CO_2$ incubator, and cultured at 37°C for 24 h. Then, composition solutions prepared in Examples 1-3, Examples 5-6, and Comparative Example 4 (formulated with a complete culture medium, at a concentration of 200 $\mu$M) were added into the cell culture medium. Meanwhile, a control group (the composition solutions were replaced with a complete culture medium) and a blank group (only a complete culture medium) were set, and 3 replicate wells were set for each group. Continued culture was performed under 5% $CO_2$ at 37°C for 24 h. Then, 25 $\mu$L of MTT was added for continued culture for 2-4 h, the culture medium was rinsed off, DMSO was added at 150 $\mu$L/well, and shaking was performed for 10 min. Absorbance values at a wavelength of 570 nm were measured using a microplate reader, and cell survival rates were calculated. Results are shown in Table 2.

Table 2

| Group | Cell survival rate (%) |
|---|---|
| Example 1 | 150.43 |
| Example 2 | 143.76 |
| Example 3 | 142.54 |
| Example 5 | 127.43 |
| Example 6 | 125.65 |
| Comparative Example 4 | 95.73 |

From data in Table 2, it can be seen that the cell survival rates of the compositions prepared in Examples 1-3 and Examples 5-6 are all above 100%, indicating that the compositions have extremely low toxicity and may even promote cell growth. A comparison with Comparative Example 4 fully indicates that the compositions involved in the present invention can effectively reduce the toxicity of artemisinic acid and have detoxification and proliferation effects on the cells.

By comparing Examples 1-3 with Examples 5-6, it can be found that compared with the combinations of the artemisinic acid and the nicotinamide or the artemisinic acid and the glutathione, the combinations of the artemisinic acid, the nicotinamide, and the glutathione can reduce the toxicity of artemisinic acid more effectively.

Test Example 3

This test example evaluates biological activities (cell experiment) of the compositions prepared in Examples 1-11 and Comparative Examples 1-6.

B16F10 cells in a state of an exponential growth phase were selected, digested with trypsin, and inoculated into a 96-well plate at a cell density of $7 \times 10^4$ cells/mL at 2 mL/well. After adherent culture overnight, a culture medium was replaced, the cells were treated with composition solutions prepared in Examples 1-11 and Comparative Examples 1-6 (formulated with a complete culture medium, at a concentration of 200 $\mu$M) for 48 h, and 3 replicate wells were set for each sample. After the culture medium was discarded, the cultured cells were rinsed once with PBS. Cell culture dishes were placed on an ice plate, 330 $\mu$L of a non-denaturing cell lysis buffer (containing 1 mM of PMSF) was added into each dish for lysis at 4°C for 20 min, and the cells were collected. Centrifugation was performed at 13,000 r/min for 10 min to obtain melanin precipitates at bottoms of centrifuge tubes. 330 $\mu$L of NaOH (containing 10% of DMSO) was added for vortexing to facilitate complete lysis, the cells were placed in a metal bath for lysis at 80°C for 2 h to fully dissolve the melanin precipitates, vortexing was performed for uniform mixing, and 200 $\mu$L of a melanin dissolution solution was added into each well of the 96-well plate.

Meanwhile, a blank group (ultrapure water) and a control group (the composition solutions were replaced with a complete culture medium) were set, and OD values at 405 nm were measured. Results are shown in Table 3.

$$\text{Relative content of melanin} = \frac{\text{OD value of sample group - OD value of blank group}}{\text{OD value of control group - OD value of blank group}} \times 100\%$$

Table 3

| Group | Relative generation amount of melanin (%) |
|---|---|
| $\alpha$-arbutin | 76.43 |
| Example 1 | 42.60 |
| Example 2 | 54.58 |
| Example 3 | 55.70 |
| Example 4 | 67.43 |
| Example 5 | 64.43 |
| Example 6 | 75.23 |
| Example 7 | 66.43 |
| Example 8 | 63.93 |
| Example 9 | 121.84 |
| Example 10 | 84.32 |
| Example 11 | 84.23 |
| Comparative Example 1 | 62.32 |
| Comparative Example 2 | 158.82 |
| Comparative Example 3 | 142.73 |
| Comparative Example 4 | 63.23 |
| Comparative Example 5 | 73.71 |
| Comparative Example 6 | 69.65 |

From data in Table 3, it can be seen that the compositions involved in the present invention can effectively inhibit the generation of melanin.

By comparing Example 1, Example 5, and Example 7 with Comparative Example 1, it can be found that the combinations of the artemisinic acid with the nicotinamide and the glutathione play a synergistic effect in inhibiting the generation of melanin.

By respectively comparing Example 1 with Examples 8-9, comparing Examples 4-5 with Example 10, and comparing Examples 6-7 with Example 11, it can be found that when the artemisinic acid, the nicotinamide, and the glutathione in the compositions are selected at specific ratios, the generation of melanin can be further inhibited.

By comparing Example 1 with Comparative Examples 2-3, it can be found that compared with the artemisinin and the Artemisia annua extract, the artemisinic acid used can achieve a better effect of inhibiting the generation of melanin.

Test Example 4

This test example determines water solubility in Examples 1, 5, and 7, and Comparative Examples 4, 5, and 6.

10 mg of artemisinic acid, nicotinamide, glutathione, and the compositions in Examples 1, 5, and 7 were weighed and dissolved in 5 mL of a solvent, respectively, and whether they were dissolved or had granular precipitates was observed. Results are shown in FIG. 1 (1 refers to an artemisinic acid aqueous solution, 2 refers to an artemisinic acid solution in DMSO, 3 refers to a nicotinamide aqueous solution, 4 refers to a glutathione aqueous solution, and 5-7 refer to aqueous solutions of the compositions of Examples 5, 7, and 1, respectively). The artemisinic acid has a large number of precipitates in water, and when the artemisinic acid is compounded with the nicotinamide or the glutathione, or when the three are added simultaneously, the solutions do not have granular precipitates and the solutions are in a clear state, indicating that the compositions have good water solubility. By compounding the artemisinic acid with the nicotinamide and/or the glutathione in the present invention, the problem of poor water solubility of the artemisinic acid is solved.

Test Example 5

This test example conducts a human body whitening experiment using the cream prepared in Application Example 1.

To eliminate other interferences and verify a whitening effect of the composition, 8 volunteers with healthy skin and no history of allergy to skin care products were selected by the applicant. Arm areas were selected as test sites with suitable area sizes, the cream with the whitening effect prepared in Application Example 1 was applied to half areas, and a blank control cream (blank control cream prepared without adding the composition of Example 1, with other ingredient proportions and a preparation method consistent with those in Application Example 1) was applied to the other half areas. The creams were respectively used for 1 time in the morning and in the evening every day, and were continuously used for 4 weeks, where the creams were prevented from being rinsed off by water after application. Before use and after every 1 week, melanin contents in the skin were measured using a skin melanin tester, respectively. Results are shown in Table 4.

Table 4

| Subject number | Sample used | Week 0 | Week 1 | Week 2 | Week 3 | Week 4 |
|---|---|---|---|---|---|---|
| 1 | Application | 126.23 | 121.43 | 115.43 | 109.42 | 100.43 |
| | Blank control | 124.43 | 123.54 | 120.43 | 122.65 | 123.76 |
| 2 | Application | 112.54 | 109.43 | 106.43 | 97.67 | 91.43 |
| | Blank control | 113.32 | 112.89 | 113.45 | 115.64 | 114.54 |
| 3 | Application | 121.43 | 114.53 | 105.92 | 100.13 | 97.32 |
| | Blank control | 122.65 | 123.43 | 122.76 | 121.76 | 123.09 |
| 4 | Application | 113.54 | 108.43 | 102.43 | 100.43 | 95.43 |
| | Blank control | 114.23 | 113.43 | 115.43 | 112.76 | 113.65 |
| 5 | Application | 123.43 | 120.53 | 112.92 | 107.13 | 102.32 |
| | Blank control | 122.65 | 122.43 | 124.76 | 121.43 | 121.39 |
| 6 | Application | 112.54 | 110.43 | 108.43 | 100.43 | 98.43 |
| | Blank control | 112.23 | 113.43 | 112.43 | 115.76 | 114.65 |

(continued)

| Subject number | Sample used | Week 0 | Week 1 | Week 2 | Week 3 | Week 4 |
|---|---|---|---|---|---|---|
| 7 | Application | 127.23 | 120.43 | 116.43 | 105.42 | 97.43 |
| | Blank control | 124.73 | 123.47 | 122.43 | 123.16 | 121.48 |
| 8 | Application | 114.54 | 109.45 | 100.43 | 97.48 | 90.29 |
| | Blank control | 112.32 | 113.89 | 112.39 | 114.74 | 112.54 |

From data in Table 4, it can be seen that the cream in Application Example 1 has a better ability to reduce pigment deposition. After use by the subjects for four weeks, the melanin contents in the arm skin of the subjects are generally decreased by approximately 20, and the melanin contents fluctuate up and down at initial values and do not have obvious changes after the blank control cream is used under same conditions. It is indicated that the composition prepared in Example 1 has an obvious effect of reducing the pigment deposition.

The applicant declares that the technical solutions of the present invention are illustrated through the above examples, but the present invention is not limited to the above examples, that is, not meaning that the present invention needs to rely on the above examples to be implemented. Those skilled in the art should understand that any improvements to the present invention, equivalent substitutions of various raw materials in a product of the present invention, addition of auxiliary ingredients, and selections of specific manners, all fall within the scope of protection and the scope of disclosure of the present invention.

The preferred embodiments of the present invention have been described in detail above. However, the present invention is not limited to specific details in the above embodiments. Various simple variations to the technical solutions of the present invention can be made within the scope of a technical concept of the present invention, and these simple variations are all within the scope of protection of the present invention.

In addition, it should be noted that various specific technical features described in the above specific embodiments may be combined in any suitable manner without a conflict, and to avoid unnecessary repetitions, various possible combination manners are not further described in the present invention.

## Claims

1. A composition with a whitening effect, **characterized in that** the composition comprises artemisinic acid, and further comprises nicotinamide and/or glutathione.

2. The composition with a whitening effect according to claim 1, **characterized in that** the composition comprises the artemisinic acid and the nicotinamide, and a mass ratio of the artemisinic acid to the nicotinamide is 1:(1-5).

3. The composition with a whitening effect according to claim 1, **characterized in that** the composition comprises the artemisinic acid and the glutathione, and a mass ratio of the artemisinic acid to the glutathione is 1:(5-10).

4. The composition with a whitening effect according to claim 1, **characterized in that** the composition comprises the artemisinic acid, the nicotinamide, and the glutathione, and a mass ratio of the artemisinic acid, the nicotinamide, and the glutathione is 1:(1-5):(3-7).

5. A skin care product, **characterized in that** the skin care product comprises the composition with a whitening effect according to any one of claims 1-4.

6. The skin care product according to claim 5, **characterized in that** a mass percentage content of the composition with a whitening effect in the skin care product is 1-20%.

7. The skin care product according to claim 5 or 6, **characterized in that** the skin care product further comprises any one or a combination of at least two of an antioxidant, a preservative, a humectant, a solubilizer, a chelating agent, an emulsifier, an emollient, a thickener, a surfactant, and a pH regulator.

8. The skin care product according to claim 7, **characterized in that** the humectant is selected from any one or a combination of at least two of glycerol, propylene glycol, 1,2-hexanediol, hyaluronic acid, sodium hyaluronate, panthenol, sodium pyrrolidonecarboxylate, polyglycerol, butanediol, dipropylene glycol, sorbitol, polyethylene glycols, Lubrajel oil, biosaccharide gum, pentanediol, betaine, or trehalose.

9. The skin care product according to claim 7 or 8, **characterized in that** the emollient is selected from any one or a combination of at least two of squalane, meadowfoam seed oil, polydimethylsiloxane, a polydimethylsiloxane cross-linking product, shea butter, jojoba seed oil, macadamia ternifolia seed oil, grape seed oil, olive fruit oil, cetyl palmitate, ethylhexyl palmitate, tocopheryl acetate, hydrogenated polyisobutene, C10-18 fatty acid triglycerides, caprylic/capric triglyceride, cyclohexasiloxane, decamethylcyclopentasiloxane, dimethiconol, caprylyl methicone, dicaprylyl carbonate, isododecane, or pentaerythityl tetraisostearate.

10. The skin care product according to any one of claims 5-9, **characterized in that** the skin care product comprises a toner, an essence, an emulsion, a cream, a gel, a facial mask, or a facial cleanser.

FIG. 1

# EP 4 763 187 A1

## INTERNATIONAL SEARCH REPORT

| International application No. |
| --- |
| **PCT/CN2024/129201** |

| **A.** | **CLASSIFICATION OF SUBJECT MATTER** |
| --- | --- |

A61K8/36(2006.01)i; A61K8/67(2006.01)i; A61K8/64(2006.01)i; A61Q19/02(2006.01)i; A61Q19/00(2006.01)i

According to International Patent Classification (IPC) or to both national classification and IPC

| **B.** | **FIELDS SEARCHED** |
| --- | --- |

Minimum documentation searched (classification system followed by classification symbols)

ICP: A61K A61Q

Documentation searched other than minimum documentation to the extent that such documents are included in the fields searched

Electronic data base consulted during the international search (name of data base and, where practicable, search terms used)

CNTXT, ENTXTC, WPABS, DWPI, CJFD, CNKI, 万方, WANFANG, ISI Web of Science, STN on the Web: 云南贝泰妮生物科技集团股份有限公司, 云南特色植物提取实验室有限公司, 王懿椿, 王飞飞, 曲丽萍, 沈艳珍, 王祖定, 吴琼粉, 闫丽娟, 杨兆祥, 马骁, 郭振宇, 青蒿, 青蒿酸, 蒿酸, 阿替米星酸, 烟碱酰胺, 烟酸胺, 烟酰胺, 菸酰胺, 尼克酰胺, 3-吡啶甲酰胺, pp因子, 维生素B3, 维生素PP, 谷胱甘肽, 谷胱苷肽, 美白, 黑色素, 抗氧化, 协同, artemisi+, artemisinic aicd, ni???inamide, VB3, VPP, +glutathione, GSH, skin 2d white+, melanog?+, 80286-58-4, 34441-14-0, 70-18-8, 27205-41-8

| **C.** | **DOCUMENTS CONSIDERED TO BE RELEVANT** |
| --- | --- |

| Category* | Citation of document, with indication, where appropriate, of the relevant passages | Relevant to claim No. |
| --- | --- | --- |
| PX | CN 118986761 A (YUNNAN BOTANEE BIO-TECHNOLOGY GROUP CO., LTD. et al.) 22 November 2024 (2024-11-22) <br> claims 1-10 | 1-10 |
| Y | LEE, Jongsung et al. "Artemisinic Acid Inhibits Angiogenesis Through Downregulation of C/EBP Alpha-Dependent Expression of HMG-CoA Reductase Gene" <br> *Food and Chemical Toxicology*, Vol. 51, 12 October 2012 (2012-10-12), 225-230 <br> page 225, abstract | 1, 5-10 |
| A | LEE, Jongsung et al. "Artemisinic Acid Inhibits Angiogenesis Through Downregulation of C/EBP Alpha-Dependent Expression of HMG-CoA Reductase Gene" <br> *Food and Chemical Toxicology*, Vol. 51, 12 October 2012 (2012-10-12), 225-230 <br> page 225, abstract | 2-4 |
| Y | KR 20130082063 A (BIO SPECTRUM INC.) 18 July 2013 (2013-07-18) <br> claims 1-7 | 1, 5-10 |

☑ Further documents are listed in the continuation of Box C.    ☑ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| **30 May 2025** | **06 June 2025** |

| Name and mailing address of the ISA/CN | Authorized officer |
| --- | --- |
| **China National Intellectual Property Administration (ISA/CN)** <br> **China No. 6, Xitucheng Road, Jimenqiao, Haidian District, Beijing 100088** | |
| | Telephone No. |

Form PCT/ISA/210 (second sheet) (July 2022)

**INTERNATIONAL SEARCH REPORT**

| International application No. |
| --- |
| **PCT/CN2024/129201** |

**C.    DOCUMENTS CONSIDERED TO BE RELEVANT**

| Category* | Citation of document, with indication, where appropriate, of the relevant passages | Relevant to claim No. |
| --- | --- | --- |
| A | KR 20130082063 A (BIO SPECTRUM INC.) 18 July 2013 (2013-07-18)<br>    claims 1-7 | 2-4 |
| Y | CN 116831939 A (GUANGDONG JIDA GENETIC MEDICINE ENGINEERING RESEARCH CENTER CO., LTD. et al.) 03 October 2023 (2023-10-03)<br>    description, paragraph [0006] | 1, 5-10 |
| A | CN 116831939 A (GUANGDONG JIDA GENETIC MEDICINE ENGINEERING RESEARCH CENTER CO., LTD. et al.) 03 October 2023 (2023-10-03)<br>    description, paragraph [0006] | 2-4 |
| A | CN 111012725 A (YUNNAN EVERGREEN BIOLOGICAL CORP. et al.) 17 April 2020 (2020-04-17)<br>    description, paragraphs [0006] and [0021] | 1-10 |
| A | CN 114286663 A (AIBI BIOTECHNOLOGY CO., LTD.) 05 April 2022 (2022-04-05)<br>    description, paragraphs [0037]-[0074] | 1-10 |

Form PCT/ISA/210 (second sheet) (July 2022)

**INTERNATIONAL SEARCH REPORT**
Information on patent family members

International application No.

**PCT/CN2024/129201**

| Patent document cited in search report | | | Publication date (day/month/year) | Patent family member(s) | | | Publication date (day/month/year) |
|---|---|---|---|---|---|---|---|
| CN | 118986761 | A | 22 November 2024 | None | | | |
| KR | 20130082063 | A | 18 July 2013 | KR | 101452061 | B1 | 22 October 2014 |
| CN | 116831939 | A | 03 October 2023 | None | | | |
| CN | 111012725 | A | 17 April 2020 | None | | | |
| CN | 114286663 | A | 05 April 2022 | WO | 2021025529 | A1 | 11 February 2021 |
| | | | | US | 2023018245 | A1 | 19 January 2023 |
| | | | | KR | 102186696 | B1 | 04 December 2020 |
| | | | | JP | 2022543701 | A | 13 October 2022 |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 202411313668